# EUROPÄISCHE PATENTSCHRIFT

(11) **EP 0 728 743 B1**
(45) Veröffentlichungstag und Bekanntmachung des Hinweises auf die Patenterteilung: **30.10.2002**
(21) Anmeldenummer: 96102127.6
(22) Anmeldetag: 14.02.1996
(51) Int. Cl.: C07D 209/88

(54) **(3-Amino)-tetrahydrocarbazol-propansäureester**
3-Amino-tetrahydrocarbazol propanoic acid esters
Esters de l'acide 3-amino-tétrahydrocarbazol propylique

(30) Priorität: 27.02.1995 DE 19506739
(43) Veröffentlichungstag der Anmeldung: 28.08.1996
(73) Patentinhaber: BAYER AG, 51368 Leverkusen (DE)
(72) Erfinder: Samaan, Samir, Prof. Dr., D-42113 Wuppertal (DE); Lanz, Joachim, Dr., D-42115 Wuppertal (DE); Naab, Paul, Dr., D-42287 Wuppertal (DE); Rosentreter, Ulrich, Dr., D-42349 Wuppertal (DE)

(56) Entgegenhaltungen:
- EP-A- 0 138 491
- EP-A- 0 425 906
- EP-A- 0 451 634
- DE-A- 2 652 447
- CHEMICAL ABSTRACTS, vol. 86, no. 22, 30.Mai 1977 Columbus, Ohio, US; abstract no. 156872x, XP002006258 & SEN'I GAKKAISHI, Bd. 33, Nr. 3, 1977, Seiten t109-t114, M. SHIMADA ET AL.:
- CHEMICAL ABSTRACTS, vol. 102, no. 16, 22.April 1985 Columbus, Ohio, US; abstract no. 132561t, XP002006259 & KHIM. TEKHNOL. ELEMENTOORG. SOEDIN. POLIM., 1984, Seiten 32-34, E.N. ZIL'BERMAN ET AL.:

## Beschreibung

Die Erfindung betrifft [3-Amino]-tetrahydrocarbazol-propansäureester, Verfahren zu ihrer Herstellung und deren Verwendung zur Synthese von Wirkstoffen.

In der Europäischen Patentanmeldung EP-242 518 werden thromboxanantagonistische Wirkstoffe der folgenden allgemeinen Formel offenbart.

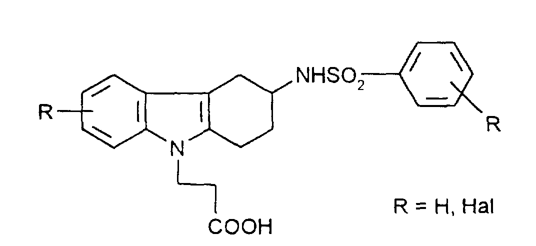

In dieser Anmeldung wird auch die Synthese dieser Wirkstoffe beschrieben: Ausgehend vom Amin (A), das zunächst in das Sulfonamid (B) überführt wird, wird durch Addition von Acrylnitril die Cyanoethyl-substituierte Verbindung (C) hergestellt, woraus durch Hydrolyse der Wirkstoff (D) erhalten wird.

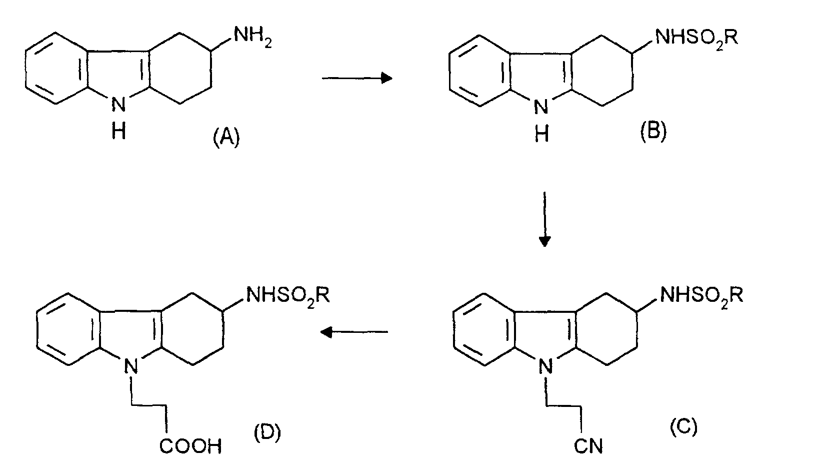

Dieses Herstellungsverfahren hat jedoch entscheidende Nachteile: Ganz abgesehen davon, daß hier das krebserregende Acrylnitril eingesetzt wird [(B) zu (C)] hat dieses Verfahren weitere Nachteile. So ist durch die Sulfonylgruppe die N-H-Bindung in der 3-Position in (B) so acidifiziert, daß auch diese Stelle von Acrylnitril angegriffen wird, was zu Nebenprodukten führt. Auch die notwendigen drastischen Bedingungen zur Verseifung des Nitrils zur Carbonsäure mindern Qualität und Ausbeute des Endproduktes.

Es besteht daher ein großes Bedürfnis nach einer Möglichkeit, die bekannten Wirkstoffe ohne Verwendung von Acrylnitril jedoch in guter Ausbeute und von guter Qualität (weitgehend frei von Nebenprodukten) herzustellen.

Die Erfindung betrifft [3-Amino]-tetrahydrocarbazol-propansäureester der allgemeinen Formel (I) 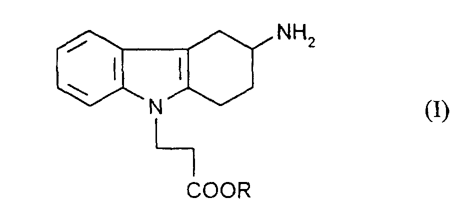 in welcher
- R: für geradkettiges oder verzweigtes Alkyl mit bis zu 10 Kohlenstoffatomen steht
und deren Salze.
- R: steht bevorzugt für geradkettiges oder verzweigtes Alkyl mit 6, besonders bevorzugt mit 4 Kohlenstoffatomen.

Die erfindungsgemäßen Verbindungen können auch in Form ihrer Salze vorliegen. Im Rahmen der Erfindung sind dies im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure oder Salze mit organischen Carbon- oder Sulfonsäuren wie beispielsweise Essigsäure, Malonsäure, Maleinsäure, Fumarsäure, Äpfelsäure, Zitronensäure, Weinsäure, Milchsäure, Benzoesäure oder Methansulfonsäure, Ethansulfonsäure, Phenylsulfonsäure, Toluolsulfonsäure, Trifluormethylsulfonsäure oder Naphthalindisulfonsäure.

Außerdem können die erfindungsgemäßen Verbindungen in verschiedenen stereoisomeren Formen existieren. Sowohl die einzelnen Isomeren (Enantiomeren/-Diastereomere) als auch deren Mischungen sind Gegenstand der Anmeldung.

Außerdem wurden zwei Verfahren zur Herstellung der Verbindungen der allgemeinen Formel (I) gefunden, dadurch gekennzeichnet, daß man

### Verfahren A

Tetrahydrocarbazole der allgemeinen Formel (II) 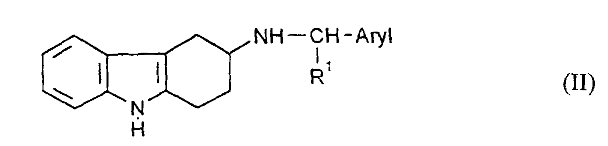 in welcher
- R¹: für Wasserstoff oder Methyl steht,
und
- Aryl: für gegebenenfalls substituiertes Naphthyl oder Phenyl steht,
mit Acrylestern der Formel (III)

H₂C=CH-COOR (III)

gegebenenenfalls in inerten Lösungsmitteln, gegebenenfalls in Anwesenheit von Hilfsmitteln umsetzt und anschließend die Gruppe -CH(R¹)-Aryl abspaltet, oder

### Verfahren B

daß man Tetrahydrocarbazole der Formel IV 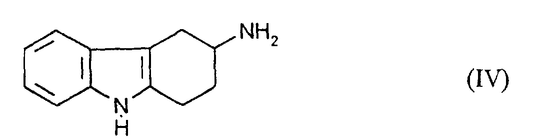 gegebenenfalls in inerten Lösungsmitteln, gegebenenfalls in Anwesenheit von Hilfsmitteln mit Acrylestern (III) umsetzt.

Da Verbindungen der Formel (IV) auch aus den Tetrahydrocarbazolen der allgemeinen Formel (II) durch Abspaltung der benzylischen Gruppen hergestellt werden können, lassen sich beide Verfahren beispielhaft durch folgendes Formelschema erläutern:

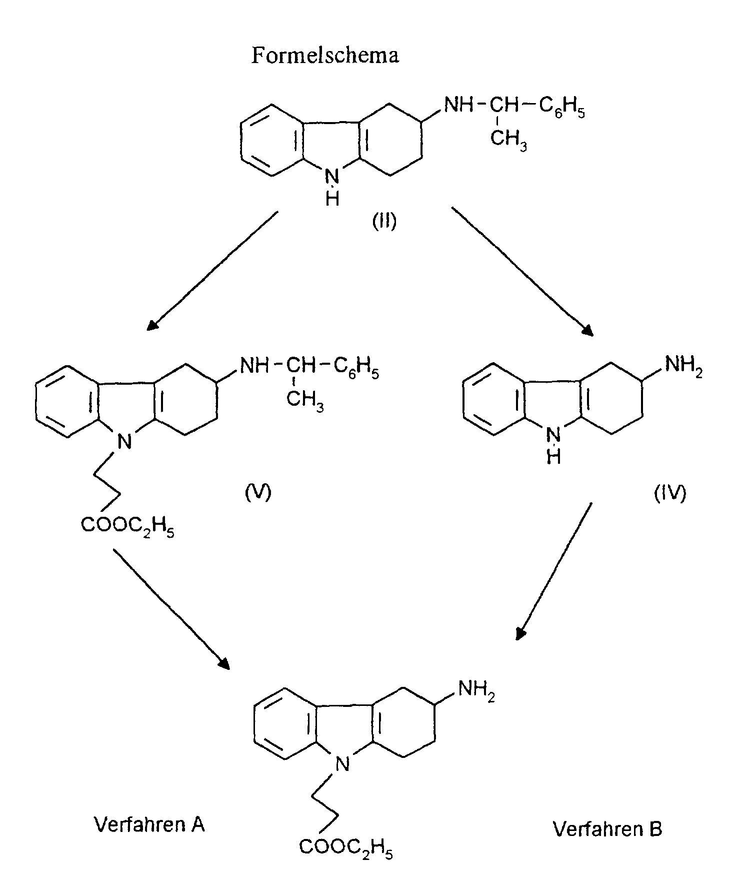

Lösemittel für das erfindungsgemäße Verfahren können Wasser und organische Lösemittel sein, die sich unter den Reaktionsbedingungen nicht verändern. Hierzu gehören bevorzugt polar aprotische Lösungsmittel wie Dimethylformamid, N-Methylpyrrolidon, Dimethylsulfoxid oder Ether wie Diethylether, Tetrahydrofuran, Dioxan, Glykolmono- oder -dimethylether, Kohlenwasserstoffe wie Benzol, Toluol, Xylol, Cyclohexan, Hexan oder Erdölfraktionen sowie chlorierte Kohlenwasserstoffe wie beispielsweise Dichlormethan, Trichlormethan, Tetrachlormethan oder Dichlorethylen. Weiterhin können Ketone wie Aceton, Methylethylketon oder Methylisobutylketon verwendet werden.

Die erfindungsgemäßen Verfahren werden im allgemeinen in einem Temperaturbereich von -50°C bis +120°C, bevorzugt von 0 bis 80°C durchgeführt.

Die erfindungsgemäßen Verfahren werden im allgemeinen bei Normaldruck durchgeführt, es ist aber auch möglich, bei Unterdruck oder Überdruck zu arbeiten.

Der Acrylester der Formel (III) wird im allgemeinen in einer Menge von 1 bis 20, bevorzugt 1 bis 10, besonders bevorzugt in einer Menge von 1 bis 5 Mol, berechnet auf das Ausgangsprodukt (II) eingesetzt.

Als Hilfsmittel werden im allgemeinen Basen verwendet. Geeignet sind hierbei die üblichen basischen Verbindungen wie Alkali- und Erdalkalihydroxide wie beispielsweise Lithium-, Natrium- oder Kaliumhydroxid oder Alkalialkoholate wie beispielsweise Natriummethanolat oder Natriumethanolat oder Kaliummethanolat oder Kalium-tert.-butylat. Bevorzugte Hilfsmittel sind Natrium- oder Kaliumhydroxid.

Bevorzugt wird das erfindungsgemäße Verfahren in einem Zweiphasensystem, bestehend aus Wasser und einem nicht wasserlöslichen organischen Lösemittel in Gegenwart eines geeigneten Phasentransferkatalysators durchgeführt. Bevorzugte Lösemittelsysteme sind Mischungen aus Wasser mit Benzol, Toluol, Xylol, Dichlormethan, Tetrahydrofuran oder Methyl-isobutylketon. Ebenso bevorzugt ist ein fest-flüssig-Zweiphasensystem in dem die Hilfsmittel wasserfrei in den angegebenen Lösungsmittel eingespeist werden.

Bevorzugte Phasentransferkatalysatoren sind beispielsweise Tetrabutyl-ammoniumiodid, Tetrabutyl-ammoniumbromid, Tetrabutyl-ammoniumchlorid, Tributyl-methylphosphoniumbromid, Trimethyl-C₁₃-C₁₅-alkylammoniumchlorid, Trimethyl-C₁₃-C₁₅-alkylammoniumbromid, Dibenzyl-dimethyl-ammoniumethylsulfonat, Dimethyl-C₁₂-C₁₅-Alkylbenzylammoniumchlorid, Dimethyl-C₁₂-C₁₄-alkyl-benzylammmoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Besonders bevorzugt als Phasentransferkatalysatoren sind Benzyl-triethylammoniumchlorid, Tetrabutylammoniumbromid.

Das Abspalten der Gruppe -CH(CH₃)C₆H₅ erfolgt nach üblichen Methoden mittels Wasserstoff unter Pd-Katalyse unter Druck oder mit Salzen der Ameisensäure, z.B. Ammoniumformiat unter Pd-Katalyse drucklos [vgl. u.a. Arzneim. Forsch./Drug Res. 3a 1519 (1989)].

Die erfindungsgemäßen Verbindungen eignen sich als Zwischenverbindungen zur Herstellung von den aus EP 242 518 bekannten Wirkstoffen. Die Verbindungen der allgemeinen Formel (I) können nach Sulfonierung an der 3-Aminogruppe in einfacher Weise zu den gewünschten Carbonsäuren hydrolysiert werden. Der Vorteil hierbei ist, daß bei der Hydrolyse der Alkoxycarbonylverbindung zur Carboxyverbindung bereits bei Raumtemperatur mit ca. 10 %iger Natronlauge abläuft (2 Stunden bei 50°C) im Gegensatz zur entsprechenden Cyanoverbindung, die 16 Stunden mit Kalilauge bei 100°C für die Verseifung benötigt [vgl. U.Rosentreter et. al, Arzneim. Forsch. Drug Res. 39, 1519 (1981)].

Außerdem können die erfindungsgemäßen Verbindungen auf einfache Art und Weise als enantiomere Verbindungen hergestellt werden, womit die Zugänglichkeit enantiomeren reiner Endprodukte wesentlich vereinfacht wird.

Die Ausgangsverbindung der Formel (II) ist bekannt (EP 242 518).

Die Acrylester der Formel (III) sind ebenfalls bekannt.

### Beispiel 1

### Ethyl-3-[3R-(1-phenylethyl)-amino-1,2,3,4-tetrahydrocarbazol-9-yl]-propionat

0,127 Mol 3R-(1-phenylethyl)-amino-1,2,3,4-tetrahydrocarbazol (als Hydrochlorid oder Sulfat) werden in ca. 125 ml Toluol suspendiert, mit 80 ml ca. 10 %iger Natronlauge versetzt und ca. 30 Minuten unter Rückfluß (ca. 84°C) gut gerührt. Bei 40°C werden 20 g Kieselgur gegeben und anschließend filtriert. Die toluolische Phase des Filtrats wird abgetrennt und durch Abdestillieren vom Wasser befreit.

Bei ca. 50°C werden zur toluolischen Lösung 0,094 Mol wasserfreie Pottasche, 0,006 Mol Benzyl-triethylammoniumchlorid und 0,156 Mol Ethylacrylat gegeben. Es wird 90 Minuten bei 80°C gerührt, mit Aktivkohle versetzt, abgekühlt und filtriert. Das Filtrat wird am Rotationsverdampfer eingedampft und der Rückstand aus Isopropanol umkristallisiert.

Ausbeute: 81-85 % d.Th., Gehalt (HPLC) > 98 %; d.e. > 98 %.

### Beispiel 2

### tert.-Butyl-3-[3R-(1-phenylethyl)-amino-1,2,3,4-tetrahydrocarbazol-9-yl]-propionat

0,2 Mol 3R-(1-phenylethyl)-amino-1,2,3,4-tetrahydrocarbazol-Hydrogensulfat und 0,2 Mol Tetrabutylammoniumbromid werden in 250 ml Dichlormethan gelöst und mit 130 ml konz. Natronlauge versetzt. Unter guten Rühren werden bei 10-20°C 0,22 Mol tert. Butyl-acrylat zugetropft. Nach 6-stündigem Rühren bei ca. 20°C wird mit Wasser verdünnt und die org. Phase abgetrennt. Die organische Phase wird i.Vakuum eingedampft und der Rückstand aus Isopropanol umkristallisiert. Ausb. 58 % d.Th.; Gehalt (HPLC) > 98 % (Schmp. 81°C).

### Beispiel 3

### n-Butyl-3-[3R-(1-phenylethyl)-amino-1,2,3,4-tetrahydrocarbazol-9-yl]-propionat

0,1 Mol 3R-(1-phenylethyl)-amino-1,2,3,4-tetrahydrocarbazol (Hydrochlorid) werden in Toluol mit Natronlauge (s. Beispiel 1) in die freie Base überführt. Die toluolische Lösung wird eingedampft und der Rückstand in Aceton aufgenommen. In die Acetonlösung werden 0,1 Mol wasserfreie Pottasche, 0,005 Mol Benzyltriethylammoniumchlorid und 0,12 Mol n-Butylacrylat gegeben. Man rührt 2,5 Stunden bei Rückflußtemperatur, kühlt ab, filtriert und dampft das Filtrat zur Trockne ein. Der Rückstand wird aus wenig Isopropanol umkristallisiert. Ausb. 80 % d.Th., Gehalt (HPLC: 97 %)

### Beispiel 4

### rac. Ethyl-3-[3-Amino-1,2,3,4-tetrahydrocarbazol-9-yl]-propionat (Halbsulfat)

Eine Mischung aus 0,4 Mol rac. 3-Amino-1,2,3,4-tetrahydrocarbazol, 0,3 Mol wasserfreier Pottasche, 0,08 Mol Benzyltriethylammoniumchlorid und 0,6 Mol Ethylacrylat in ca. 500 ml Methylisobutylketon wird 45 Minuten bei 90°C gerührt. Anschließend wird bei ca. 60°C abfiltriert. Das Filtrat wird i.Vakuum eingedampft, der Rückstand in ca. 500 ml Ethanol gelöst und mit ca. 20 % Schwefelsäure auf pH > 3 gestellt. Es wird 1 h bei 20°C gerührt, abfiltriert und mit Ethanol/Wasser gut gewaschen. Das Produkt wird i.Vak. getrocknet.
Ausbeute: 65-70 % d.Th.; Gehalt als freie Base (HPLC) > 78 % (theoret.:)
Elementaranalyse: Produkt x 0,5 H₂SO₄
Analog Beispiel 4 wird die enantiomerenreine Verbindung erhalten (ee > 98 % von diastereomerenreinen Ausgangsverbindung). (s. Beispiel 5).

### Beispiel 5

### Ethyl-3-[3R-amino-1,2,3,4-tetrahydrocarbazol-9-yl]-propionat (Halbsulfat)

0,06 Mol Ethyl-3-[3R-(1-phenylethyl)-amino-1,2,3,4-tetrahydrocarbazol-9-yl]-propionat (Beispiel 1) werden zu einer Suspension von 4,3 g Pd auf Kohle (10 %ig; 58,5 % wasserfeucht) in 35 ml Wasser und 80 ml Ethanol gegeben. Hierzu gibt man 0,078 Mol Ammoniumformiat und rührt 1,25 Stunden bei Rückflußtemperatur. Bei ca. 75°C tropft man 3-5 ml Essigsäure zu, kühlt auf ca. 40°C ab und filtriert vom Umgelösten. Das Filtrat wird mit ca. 20 %iger Schwefelsäure auf pH > 3 gebracht. Es wird ca. 1 Std. bei Raumtemperatur gerührt, abfiltriert, mit Wasser/Ethanol gewaschen und getrocknet. Ausbeute: 91 % d.Th.; Gehalt als freie Base (HPLC): > 78 % (ee > 98).

### Beispiel 6

### tert.-Butyl-3-[3R-amino-1,2,3,4-tetrahydrocarbazol-9-yl)-propionat:

0,08 Mol tert.-Butyl-3-[3R-(1-phenethyl)-amino-1,2,3,4-tetrahydrocarbazol-1-yl]-propionat (Beispiel 2), 24 g Pd-C-5 %ig (50 % Wasser) und 0,23 Mol Ammoniumformiat werden in einer Mischung aus 170 ml Ethanol und 70 ml Wasser bei ca. 70°C 20 Minuten gerührt. Es wird abgesaugt und zur Trockne eingedampft.
Ausbeute: 91 % d.Th.; Schmp.: 83-86°C

### Beispiel 7

### Herstellung und Reinigung von (3R)-3-(4-Fluorphenylsulfonamido)-1,2,3,4-tetrahydrocarbazol-9-yl-Propionsäure (BAY u 3405):

Zu einer Lösung von 0,515 Mol 4-Fluorbenzolsulfochlorid in 450 ml Ethylacetat werden 0,414 Mol Ethyl-3-[3R-amino-1,2,3,4-tetrahydrocarbazol-9-yl]-propionat-Halbsulfat (Beispiel 5) gegeben. Unter Rühren und Kühlen tropft man ca. 425 ml 10 %ige Natronlauge innerhalb einer Stunde (10-15°C) zu. Man rührt 30 Minuten nach und stellt den pH-Wert der Lösung mit halbkonz. Salzsäure von ca. 8 auf ca. 5 ein. Die abgetrennte organische Phase wird am Rotationsverdampfer weitgehend eingedampft. Der ölige Rückstand wird mit 350 ml 10 %iger Natronlauge versetzt und ca. 2 Stunden bei 50°C gerührt. Es wird auf 5°C abgekühlt und mit ca. 620 ml Essigester überschichtet. Unter dauernder Kühlung wird mit halbkonzentrierter Salzsäure (ca. 180 ml) ein pH-Wert von 5 eingestellt.

Die abgetrennte organische Phase wird am Rotationsverdampfer eingedampft und in ca. 650 ml Essigester aufgenommen. Zur Essigesterlösung werden 66 ml n-Butylamin in 150 ml Essigester zugetropft. Man rührt 1 Stunde bei 0°C, saugt ab und wäscht 3 mal mit je 60 ml Essigester nach. Das isolierte Butylammoniumsalz (Ausb.: quantitativ: Gehalt > 98 %) kann bei Bedarf aus Isopropanol/Essigester umkristallisiert werden. Das Salz wird in 440 ml Wasser gelöst, mit 740 ml Essigester überschichtet und unter Kühlung mit ca. 220 ml halbkonz. Salzsäure langsam versetzt. Die organische Phase wird 2 mal mit Sole gewaschen, über Natriumsulfat getrocknet und mit 60 g Kieselgel 60 versetzt. Es wird 30 Minuten gerührt, vom Trockenmittel und Kieselgel abfiltriert und der Filterrückstand 2 mal mit je 70 ml Essigester gewaschen. Das gesammelte Filtrat wird ausgedampft und aus Essigester/Isohexan kristallisiert.
Ausbeute: 85 % d.Th.; Gehalt (HPLC): 98,9 % [α]; 71°C (c=1,0, MeOH)

## Patentansprüche

1. [3-Amino]-tetrahydrocarbazol-propansäureester der allgemeinen Formel (I) 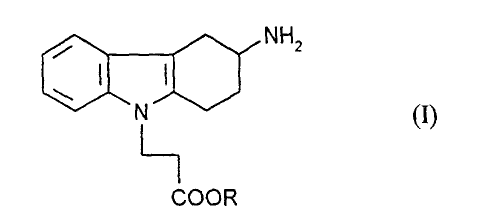 in welcher
R für geradkettiges oder verzweigtes Alkyl mit bis zu 10 Kohlenstoffatomen steht,
und deren Salze.

2. Verfahren zur Herstellung von [3-Amino]-tetrahydrocarbazol-propansäureester nach Anspruch 1, **dadurch gekennzeichnet, daß** man
das Tetrahydrocarbazol der Formel (II) 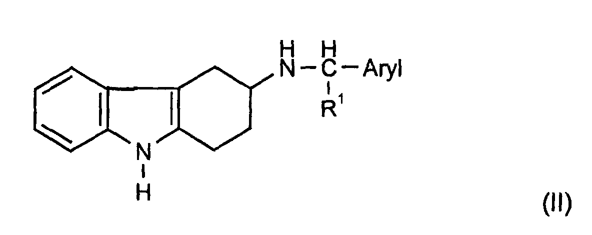 in welcher
R¹ für Wasserstoff oder Methyl steht,
und
Aryl für gegebenenfalls substituiertes Naphthyl oder Phenyl steht,
mit Acrylestern der Formel (III)
H₂C=CH-COOR (III)
gegebenenenfalls in inerten Lösungsmitteln umsetzt und anschließend die Gruppe -CH(R¹)-Aryl abspaltet.

3. Verfahren zur Herstellung von [3-Amino]-tetrahydrocarbazol-propansäureestern nach Anspruch 1, **dadurch gekennzeichnet, daß** man
Tetrahydrocarbazole der Formel IV 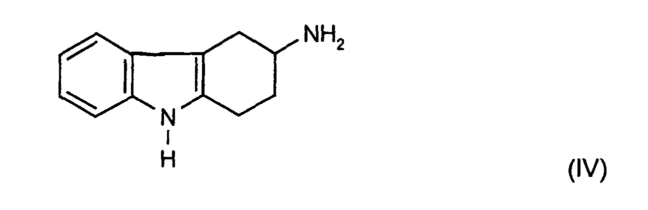 gegebenenfalls in inerten Lösungsmitteln mit Acrylestem (III)
H₂C=CH-COOR (III)
umsetzt.

4. Verfahren nach Anspruch 3, **dadurch gekennzeichnet, daß** man zuerst aus dem Tetrahydrocarbazol der Formel (II) nach Anspruch 2, die Gruppe -CH(CH₃)C₆H₅ abspaltet, und dann mit Acrylaten der Formel (III) umsetzt.

5. Verfahren nach Ansprüchen 2 bis 4, **dadurch gekennzeichnet, daß** man die Umsetzung in einem Temperaturbereich von -50°C bis +120°C durchführt.

6. Verfahren nach Ansprüchen 2 bis 5, **dadurch gekennzeichnet, daß** man die Umsetzung in einem Zweiphasen-System, bestehend aus Wasser und einem nicht-wasserlöslichen organischen Lösemittel oder in einem Zweiphasensystem bestehend aus festen Hilfsstoffen und einem organischen Lösungsmittel durchführt.

7. Verfahren nach Ansprüchen 2 bis 6, **dadurch gekennzeichnet, daß** man die Umsetzung mit Hilfe eines Phasentransferkatalysators durchführt.

8. Verwendung der [3-Amino]-tetrahydrocarbazol-propansäureester nach Anspruch 1 als Zwischenprodukte.

9. Verfahren zur Herstellung thromboxanantagonistischer Wirkstoffe der folgenden allgemeinen Formel: 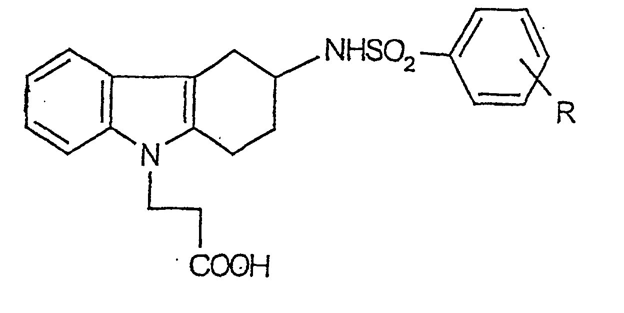 worin R gleich Wasserstoff oder Halogen darstellt,
**dadurch gekennzeichnet, daß** man entweder
[A] das Tetrahydrocarbazol der Formel (II) 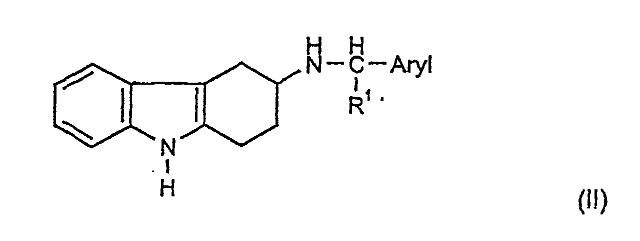 in welcher
R¹ für Wasserstoff oder Methyl steht,
und
Aryl für gegebenenfalls substituiertes Naphthyl oder Phenyl steht,
mit Acrylestern der Formel (III)
H₂C=CH-COOR (III)
worin R für geradkettiges oder verzweigtes alky mit bis zu 10 Kohlenstoffatomen steht,
gegebenenenfalls in inerten Lösungsmitteln umsetzt und anschließend die Gruppe -CH(R¹)-Aryl abspaltet, oder
[B] Tetrahydrocarbazole der Formel IV 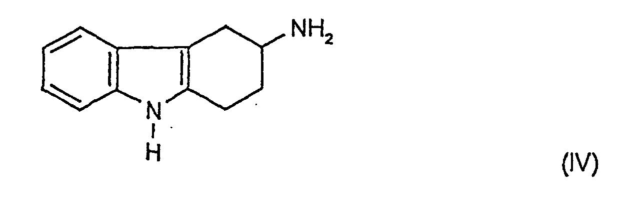 gegebenenfalls in inerten Lösungsmitteln, gegebenenfalls in Anwesenheit von Hilfsmittel mit Acrylestern (III)
H₂C=CH-COOR (III)
zu [3-Amino]-tetrahydrocarbazol-propansäure-ester der allgemeinen Formel (I) nach Anspruch 1 umsetzt
und die Verbindung der allgemeinen Formel (I) nach Sulfonierung an der 3-Aminogruppe zu den gewünschten Carbonsäuren hydrolysiert.

## Claims

1. [3-Amino]-tetrahydrocarbazole-propanoic acid esters of the general formula (I) 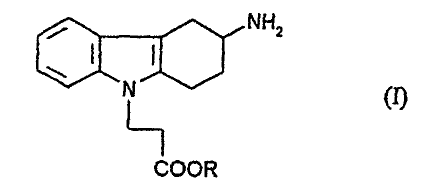 in which
R represents straight-chain or branched alkyl having up to 10 carbon atoms,
and their salts.

2. Process for the preparation of [3-amino]-tetrahydrocarbazole-propanoic acid esters according to Claim 1, **characterized in that**
the tetrahydrocarbazole of the formula (II) 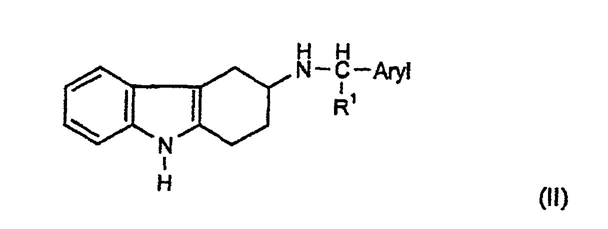 in which
R¹ represents hydrogen or methyl,
and
Aryl represents optionally substituted naphthyl or phenyl,
is reacted with acrylic esters of the formula (III)
H₂C=CH-COOR (III)
if appropriate in inert solvents, and then the group -CH(R¹)-Aryl is removed.

3. Process for the preparation of [3-amino]-tetrahydrocarbazole-propanoic acid esters according to Claim 1, **characterized in that**
tetrahydrocarbazoles of the formula (IV) 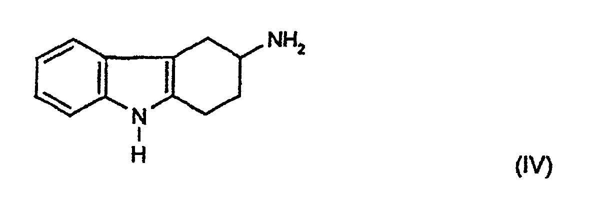 are reacted with acrylic esters (III)
H₂C=CH-COOR (III),
if appropriate in inert solvents.

4. Process according to Claim 3, **characterized in that** the group -CH(CH₃)C₆H₅ is first removed from the tetrahydrocarbazole of the formula (II) according to Claim 2, which is then reacted with acrylates of the formula (III).

5. Process according to Claims 2 to 4, **characterized in that** the reaction is carried out in a temperature range from -50°C to +120°C.

6. Process according to Claims 2 to 5, **characterized in that** the reaction is carried out in a two-phase system, consisting of water and a water-insoluble organic solvent or in a two-phase system consisting of solid auxiliaries and an organic solvent.

7. Process according to Claims 2 to 6, **characterized in that** the reaction is carried out with the aid of a phase-transfer catalyst.

8. Use of [3-amino]-tetrahydrocarbazole-propanoic acid esters according to Claim 1 as intermediates

9. Process for the preparation of thromboxane-antagonistic active compounds of the following general formula: 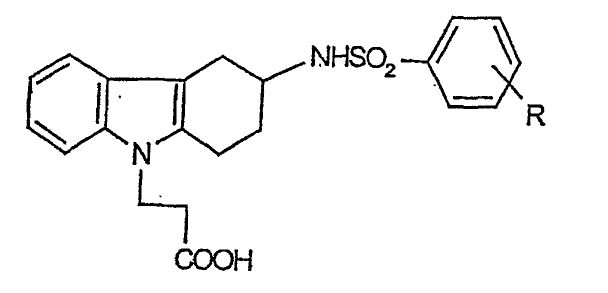 in which R is hydrogen or halogen, **characterized in that** either
[A] the tetrahydrocarbazole of the formula (II) 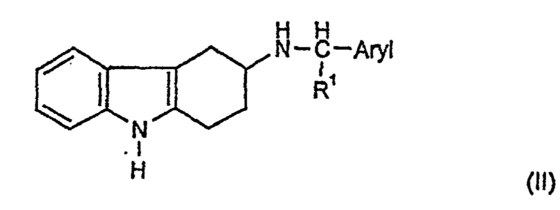 in which
R¹ represents hydrogen or methyl
and
Aryl represents optionally substituted naphthyl or phenyl,
is reacted with acrylic esters of the formula (III)
H₂C=CH-COOR (III)
in which R represents straight-chain or branched alkyl having up to 10 carbon atoms, if appropriate in inert solvents, and then the group -CH(R¹)-Aryl is removed, or
[B] tetrahydrocarbazoles of the formula IV 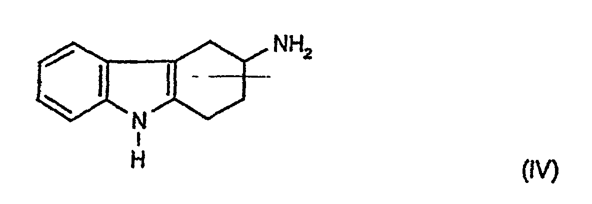 are reacted, if appropriate in inert solvents, if appropriate in the presence of auxiliaries, with acrylic esters (III)
H₂C=CH-COOR (III)
to give 3-amino-tetrahydrocarbazole-propanoic acid esters of the general formula (I) according to Claim 1
and the compound of the general formula (I) is hydrolysed to give the desired carboxylic acids after sulphonation on the 3-amino group.

## Revendications

1. Esters d'acide [3-amino]-tétrahydrocarbazole-propionique de formule générale (I) 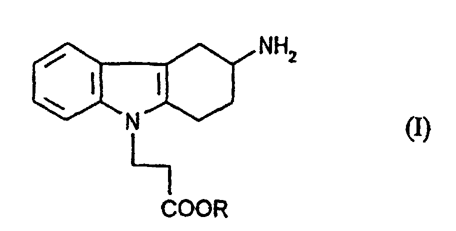 dans laquelle
R est un groupe alkyle linéaire ou ramifié ayant jusqu'à 10 atomes de carbone,
et leurs sels.

2. Procédé de production d'esters d'acide [3-amino]-tétrahydrocarbazole-propionique suivant la revendication 1, **caractérisé en ce qu'**on fait réagir un tétrahydrocarbazole de formule (II) 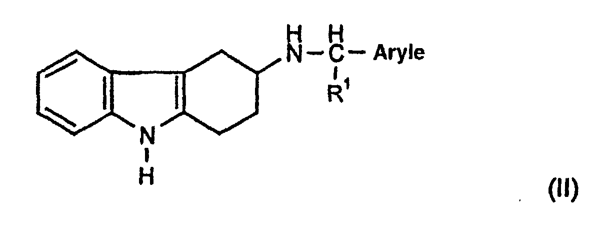 dans laquelle
R¹ est l'hydrogène ou le groupe méthyle,
et
aryle désigne un groupe naphtyle ou phényle éventuellement substitué,
avec des esters acryliques de formule (III)
H₂C=CH-COOR (III)
éventuellement dans des solvants inertes, puis on élimine le groupe -CH(R¹)-aryle.

3. Procédé de production d'esters d'acide [3-amino]-tétrahydrocarbazole-propionique suivant la revendication 1, **caractérisé en ce qu'**on fait réagir le tétrahydrocarbazole de formule (IV) 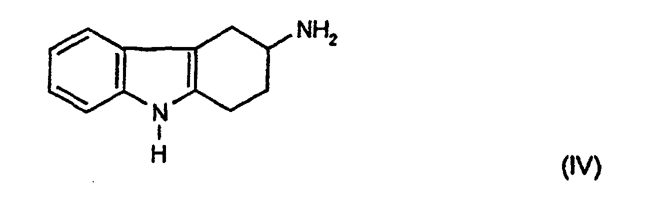 le cas échéant dans des solvants inertes, avec des esters acryliques (III)
H₂C=CH-COOR (III)

4. Procédé suivant la revendication 3, **caractérisé en ce qu'**on élimine tout d'abord le groupe -CH(CH₃)C₆H₅ du tétrahydrocarbazole de formule (II) suivant la revendication 2 puis on le fait réagir avec des acrylates de formule (III).

5. Procédé suivant les revendications 2 à 4, **caractérisé en ce qu'**on conduit la réaction dans une plage de températures de -50 °C à +120 °C.

6. Procédé suivant les revendications 2 à 5, **caractérisé en ce qu'**on conduit la réaction dans un système de deux phases constitué d'eau et d'un solvant organique non miscible à l'eau ou dans un système de deux phases constitué de substances auxiliaires solides et d'un solvant organique.

7. Procédé suivant les revendications 2 à 6, **caractérisé en ce qu'**on conduit la réaction à l'aide d'un catalyseur de transfert de phases.

8. Utilisation des esters d'acide [3-amino]-tétrahydrocarbazole-propionique suivant la revendication 1 comme produits intermédiaires.

9. Procédé de production de substances actives antagonistes du thromboxane, de formule générale suivante : 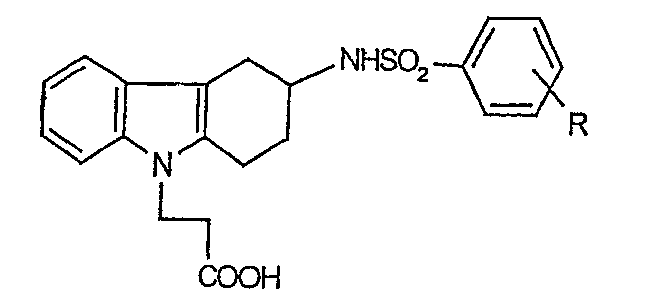 dans laquelle R représente l'hydrogène ou un halogène, **caractérisé en ce que** :
[A] on fait réagir un tétrahydrocarbazole de formule (II) 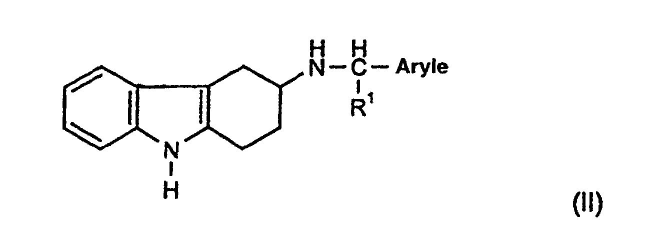 dans laquelle
R¹ représente l'hydrogène ou le groupe méthyle,
et
aryle représente un groupe naphtyle ou phényle éventuellement substitué,
avec des esters acryliques de formule (III)
H₂C=CH-COOR (III)
dans laquelle R est un groupe alkyle linéaire ou ramifié ayant jusqu'à 10 atomes de carbone,
le cas échéant dans des solvants inertes, puis on élimine le groupe -CH(R¹)-aryle, ou bien
[B] on fait réagir le tétrahydrocarbazole de formule IV 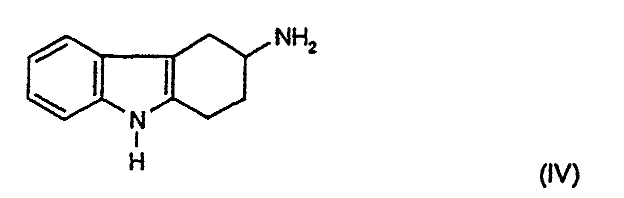 le cas échéant dans des solvants inertes, éventuellement en présence de substances auxiliaires, avec des esters acryliques (III)
H₂C=CH-COOR (III)
pour obtenir un ester d'acide [3-amino]-tétrahydrocarbazole-propionique de formule générale (I) suivant la revendication 1
et on hydrolyse le composé de formule générale (I) après sulfonation au niveau du groupe 3-amino pour obtenir les acides carboxyliques voulus.
